# EUROPÄISCHE PATENTSCHRIFT

(11) **EP 0 538 686 B1**
(45) Veröffentlichungstag und Bekanntmachung des Hinweises auf die Patenterteilung: **27.09.1995**
(21) Anmeldenummer: 92117239.1
(22) Anmeldetag: 09.10.1992
(51) Int. Cl.: C07C 31/24, C07C 29/141, C07C 47/19, C07C 45/75, C07C 45/68

(54) **Verfahren zur Herstellung von 2,2-Bis-hydroxymethyl-butandiol-(1,4)**
Process for the preparation of 2,2,-bis-hydroxymethyl-butanediol-(1,4)
Procédé de préparation de 2,2-bis-hydroxyméthyl-butanédiol-(1,4)

(30) Priorität: 22.10.1991 DE 4134771
(43) Veröffentlichungstag der Anmeldung: 28.04.1993
(73) Patentinhaber: BASF Aktiengesellschaft, 67063 Ludwigshafen (DE)
(72) Erfinder: Merger, Franz, Dr., W-6710 Frankenthal (DE); Schmidt-Radde, Martin, Dr., W-6700 Ludwigshafen (DE)

(56) Entgegenhaltungen:
- EP-A- 0 004 577
- US-A- 2 775 622
- JOURNAL OF ORGANIC CHEMISTRY. Bd. 24, 1959, EASTON US Seiten 1005 - 1006 L.P. KUHN ET AL. 'The Preparation of 2,2-Bis(hydroxymethyl)-3-butene-1-ol and 2- (Hydroxymethyl)-2-methyl-3-butene-1-ol and their Acetates and Nitrates.'

## Beschreibung

Die vorliegenden Erfindung betrifft ein Verfahren zur Herstellung von 2,2-Bis-hydroxymethyl-butandiol-(1,4) der Formel I 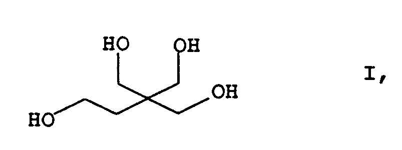
Nach J.Org.Chem. 24, 1005 (1959) entsteht bei der Coaldolisierung von 4-Hydroxybutyraldehyd mit Formaldehyd unter diskontinuierlicher Hydrierung des dabei gebildeten, rohen 2,2-Bis-hydroxymethyl-4-hydroxy-butyraldehyds mit Raney-Nickel ein dickflüssiger Sirup, aus dem die Titelverbindung nach herkömmlichen Trennmethoden nicht isolierbar ist. Zur Gewinnung von I muß das erhaltene Hydriergemisch mittels Acetanhydrid zum Tetraessigsäureester acetyliert werden, welcher isoliert werden kann und durch Umesterung mit Methanol in die Titelverbindung I überführt wird. Auf diese Weise kann 2,2-Bis-hydroxymethyl-butandiol-(1,4) I, im folgenden kurz BHB genannt, in einer Gesamtausbeute von lediglich 24 %, bezogen auf 4-Hydroxybutyraldehyd, erhalten werden. Wegen dieser unzulänglichen Ausbeute und der sehr mühsamen und aufwendigen Isolierung des Wertproduktes ist dieses Verfahren für eine industrielle Anwendung nicht geeignet.

Möglicherweise ist diese niedrige Ausbeute auf Nebenreaktionen der intermediär gebildeten Produkte 4-Hydroxy-2-hydroxymethyl-butyraldehyd und 2,2-Bis-hydroxymethyl-4-hydroxybutyraldehyd, wie Dehydratisierungs-, Retroaldol- oder Polymerisationsreaktionen zurückzuführen. Dies wurde jedoch nicht näher untersucht.

Nach EP-A 340 970 entstehen bei der Umsetzung von 4-Hydroxybutyraldehyd mit Formaldehyd unter hydrierenden Bedingungen praktisch ausschließlich 2-Methylbutandiol-(1,4) und Butandiol-(1,4). BHB wird unter diesen Bedingungen offensichtlich nicht gebildet.

Der vorliegenden Erfindung lag daher die Aufgabe zugrunde, ein Verfahren zu finden, das die wirtschaftliche Herstellung von BHB ermöglicht.

Dementsprechend wurde ein Verfahren zur Herstellung von 2,2-Bis-hydroxymethyl-butandiol-(1,4) der Formel I 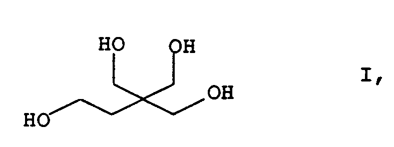
gefunden, daß dadurch gekennzeichnet ist, daß man 4-Hydroxybutyraldehyd der Formel II 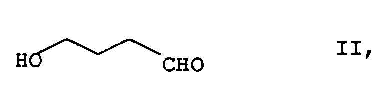
und/oder 2-Hydroxytetrahydrofuran der Formel III 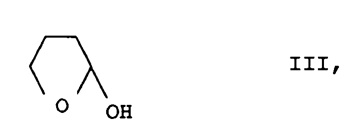
in Gegenwart eines basischen Katalysators mit Formaldehyd kondensiert, die erhaltene Reaktionsmischung anschließend katalytisch hydriert und aus dem Hydrieraustrag das 2,2-Bis-hydroxymethyl-butandiol-(1,4) isoliert.

Die dem erfindungsgemäßen Verfahren zugrunde liegenden chemischen Umsetzungen sind im folgenden Reaktionsschema veranschaulicht:

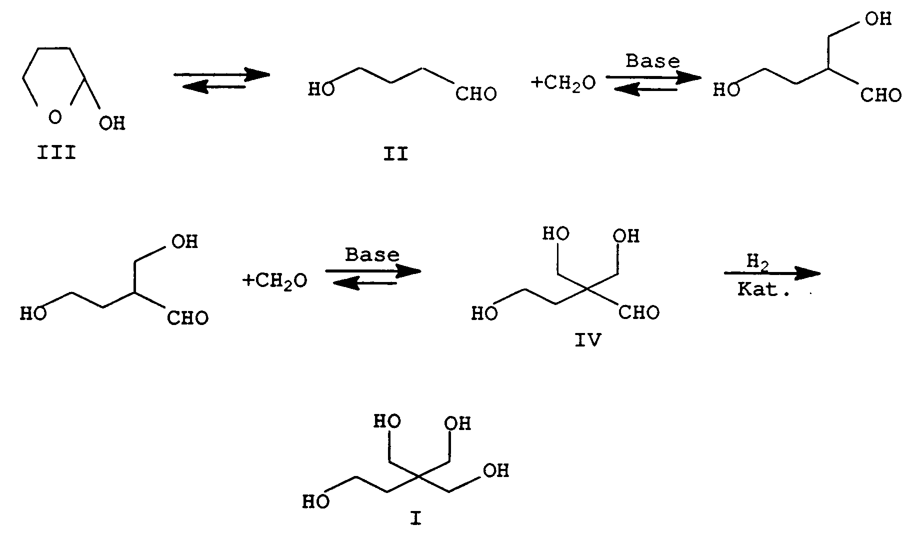

4-Hydroxybutyraldehyd II, der im Gleichgewicht mit seinem cyclischen Halbacetal 2-Hydroxytetrahydrofuran III steht, wird dabei in einer Aldolisierung mit 2 Molekülen Formaldehyd zu 2,2-Bis-hydroxymethyl-butyraldehyd IV kondensiert, welcher anschließend zu BHB hydriert wird.

Zur Aldolisierung wird der Formaldehyd bezüglich des 4-Hydroxybutyraldehyds II im allgemeinen in einem Molverhältnis von 1,5 bis 3,0, vorzugsweise in einem Molverhältnis von 1,8 bis 2,5 umgesetzt. Zweckmäßigerweise wird dazu der Formaldehyd als wässrige Lösung eingesetzt, deren Formaldehydgehalt vorteilhaft 10 bis 50 Gew.% betragen kann.

Als basische Katalysatoren können bei der Aldolisierung des Formaldehyds mit 4-Hydroxybutyraldehyd zwar alle Basen eingesetzt werden, die üblicherweise zur Katalyse von Aldolkondensationen verwendet werden, beispielsweise die Hydroxide und Carbonate der Alkali- und Erdalkalimetalle, tertiäre Amine und basische Ionenaustauscher, besonders bevorzugt werden im erfindungsgemäßen Verfahren jedoch tertiäre Amine, insbesondere tertiäre Alkylamine, benutzt.

Es können sowohl aliphatische, cycloaliphatische oder heterocycloaliphatische tertiäre Amine als Katalysatoren im erfindungsgemäßen Verfahren dienen. Zweckmäßigerweise werden tertiäre Amine mit 3 bis 20 Kohlenstoffatomen, vorzugsweise mit 3 bis 15 Kohlenstoffatomen verwendet. Besonders bevorzugte tertiäre Amine sind solche, die aus C₁- bis C₄-Alkylgruppen aufgebaut sind. Beispielhaft seien die folgenden tertiären Amine genannt: Trimethylamin, Triethylamin, Tri-n-propylamin, Tri-n-butylamin, Tri-isopropylamin, Tri-isobutylamin, Methyl-diethylamin, Methyl-diisopropylamin, Ethyl-diisopropylamin, Dimethyl-tert.-butylamin, N,N'-Tetramethyl-ethylendiamin, N,N',N''-Pentamethyl-ethylentriamin, Cyclohexyl-dimethylamin, Dicyclohexyl-methylamin, Tribenzylamin, Benzyldimethylamin, N-Methylpyrrolidin, N-Methyl-piperidin, N,N'-Dimethyl-piperazin, N,N'-Diethyl-piperazin, N-Methylmorpholin, Triethanolamin, Methyldiethanolamin, Chinuclidin, 1,4-Diazabicyclo(2,2,2)octan.

Die Basen werden in der Regel in einer Menge von 0,5 bis 25, vorteilhaft von 1,0 bis 10 Mol.%, bezogen auf 4-Hydroxybutyraldehyd, angewandt.

Die Kondensation des 4-Hydroxybutyraldehyds mit Formaldehyd wird im allgemeinen bei Temperaturen von 0 bis 100°C, vorzugsweise bei 1 bis 70°C und besonders bevorzugt, bei 1 bis 40°C durchgeführt. Zweckmäßigerweise wird die Umsetzung bei Atmosphärendruck vorgenommen, eine Arbeitsweise unter Druck, vorzugsweise unter dem Eigendruck des Reaktionssystems oder unter vermindertem Druck ist ebenfalls möglich.

Üblicherweise wird die Aldolkondensation in einem wäßrigen Reaktionsmedium durchgeführt, gewünschtenfalls können dem Reaktionsmedium aber auch noch andere, unter den Reaktionsbedingungen inerte, vorzugsweise gut mit Wasser mischbare, organische Lösungsmittel, wie Tetrahydrofuran, Dioxan oder Dimethoxyethan zugesetzt werden.

Nach beendeter Aldolkondensation und vor der Hydrierung kann der basische Katalysator gewünschtenfalls durch Zugabe einer Säure neutralisiert werden, wobei vorzugsweise mit Hilfe eines sauren Ionenaustauschers neutralisiert wird, der sich leicht durch mechanische Trennverfahren, wie Filtration oder Zentrifugation, wieder aus der Reaktionsmischung entfernen läßt. Eine solche Arbeitsweise erweist sich insbesondere bei Verwendung anorganischer Katalysatoren als zweckmäßig. Sind destillierbare basische Katalysatoren, wie tertiäre Amine, zur Aldolkondensation eingesetzt worden, so können diese gewünschtenfalls auch destillativ von der Reaktionsmischung abgetrennt werden. Es ist aber auch möglich, die nachfolgende Hydrierung in Gegenwart dieser basischen Katalysatoren auszuführen. Insbesondere bei der bevorzugten Ausführungsform des erfindungsgemäßen Verfahrens, also bei der Verwendung tertiärer Amine als Aldolisierungskatalysatoren kann die Hydrierung des Aldolisierungsprodukts direkt, d.h. ohne vorherige Abtrennung des Katalysators, vorgenommen werden.

Zur Hydrierung wird der 2,2-Bis-hydroxymethyl-4-hydroxybutyraldehyd IV vorteilhaft in Lösungen eines Gehaltes von nicht mehr als 50 Gew.% IV eingesetzt. Vor der Hydrierung setzt man dem Reaktionsgemisch aus der Aldolkondensation deshalb zweckmäßigerweise noch zusätzliches Lösungsmittel zu. Besonders bevorzugt wird das Reaktionsgemisch weiter mit Wasser verdünnt, so daß die zu hydrierende Lösung einen Wassergehalt von üblicherweise 50 bis 90 Gew.%, insbesondere von 70 bis 85 Gew.% hat.

Anstelle von Wasser können dem zu hydrierenden Reaktionsgemisch aus der Aldolkondensation auch organische Lösungsmittel, welche sich unter den Bedingungen der Hydrierung inert verhalten, zugefügt werden, vornehmlich solche, welche sich mit dem bereits in der Reaktionsmischung enthaltenen Wasser gut mischen.

Beispielhaft für solche Lösungsmittel seien Ether, wie Tetrahydrofuran, Dioxan, Dimethoxyethan, C₁- bis C₄-Alkohole, insbesondere Methanol, Ethanol, Propanol und Isopropanol, Ethylenglykol, Methoxyethanol und Ethoxyethanol genannt.

Es ist auch möglich, das Wasser aus der zu hydrierenden Reaktionsmischung ganz oder teilweise, z.B. durch Destillation, zur entfernen und anschließend ganz oder teilweise durch organische Lösungsmittel zu ersetzen. Wird das Wasser vollständig oder zum größten Teil aus der Reaktionsmischung entfernt, kann es auch durch nicht oder weniger gut mit Wasser mischbaren, organischen Lösungsmitteln, ersetzt werden. Zweckmäßigerweise verfährt man bei Verwendung oder Mitverwendung organischer Lösungsmittel so, daß man diese der zu hydrierenden Reaktionsmischung in solchen Mengen zusetzt, daß sich ein Gesamtgehalt der zu hydrierenden Reaktionsmischung von Wasser und/oder organischem Lösungsmittel von 50 bis 90 Gew.%, vorzugsweise von 70 bis 85 Gew.%, einstellt.

Die Hydrierung der so hergestellten, das Aldolkondensationsprodukt 2,2-Bis-hydroxymethyl-4-hydroxybutyraldehyd enthaltenden Lösung kann auf an sich herkömmliche Weise bei Temperaturen von im allgemeinen 70 bis 200°C und bei einem Druck von üblicherweise 1 bis 200 bar durchgeführt werden. Man hydriert bevorzugt mit Hilfe von heterogenen Katalysatoren. Dabei kann der Hydrierkatalysator in der Reaktionsmischung suspendiert oder in einem Festbett angeordnet werden. Das zu hydrierende Gemisch kann sowohl in der Sumpf- als auch in der Rieselfahrweise über das Festbett geleitet werden. Dabei werden im allgemeinen Verweilzeiten von 10 bis 300 Minuten, insbesondere von 20 bis 120 Minuten eingestellt.

Für die Hydrierung des 2,2-Bis-hydroxymethyl-4-hydroxybutyraldehyds zu BHB können prinzipiell alle üblichen Hydrierkatalysatoren verwendet werden, beispielsweise Nickel, Kobalt, Kupfer, Mangan, Molybdän, Rhenium und/oder die Platinmetalle Palladium, Platin und Ruthenium enthaltende Katalysatoren. Dabei können sowohl die reinen Metalle, feinverteilt oder in Form von Netzen oder anderen Gebilden großer Oberfläche, eingesetzt werden, als auch Katalysatoren, die mehrere dieser Metalle enthalten. Die Hydrierkatalysatoren können als Vollkatalysatoren oder in Form von Trägerkatalysatoren eingesetzt werden. Es können an sich übliche Trägermaterialien für diese Trägerkatalysatoren verwendet werden, wie Siliciumdioxid, Aluminiumoxide, Zirkondioxid, Titandioxide, Aktivkohle, Bariumsulfat, Bariumcarbonat, Calciumcarbonat und ähnliche. Beispielhaft sei auf die Hydrierkatalysatoren, die in R.L. Augustine, Catalytic Hydrogenation, Kapitel 3, Marcel Dekker, Inc., New York 1965, EP-A 335 222, DE-A 12 57 753, US-A 3 449 445, DE-A 23 21 101, EP-A 44 444 und DE-A 39 04 083 gelehrt werden, hingewiesen. Vorzugsweise werden im erfindungsgemäßen Verfahren Kupfer- und/oder Kobalt-haltige Katalysatoren und/oder Rutheniumkatalysatoren, insbesondere Ruthenium auf Aluminiumoxid, eingesetzt. Besonders bevorzugte Kupfer-und/oder Kobalt-haltige Katalysatoren sind die Katalysatoren gemäß DE-A 23 21 101, EP-A 44 444 und DE-A 39 04 083.

Sowohl die Aldolkondensation als auch die Hydrierung können diskontinuierlich oder kontinuierlich in an sich herkömmlichen Reaktoren durchgeführt werden.

Aus dem Hydriergemisch kann das Wertprodukt BHB auf einfache Weise, nach an sich herkömmlichen Methoden isoliert werden, beispielsweise durch die Extraktion einer wäßrigen Hydrierlösung mittels schwer in Wasser löslichen, organischen Lösungsmitteln, wie schwerlöslichen Alkoholen, Ketonen, Ethern, Estern oder halogenierten Kohlenwasserstoffen oder durch Kristallisation. Bevorzugt wird BHB destillativ gereinigt und isoliert. Bei der Destillation kann auch das gegebenenfalls als Katalysator verwendete tertiäre Amin wiedergewonnen und gewünschtenfalls in die Stufe der Aldolkondensation zurückgeführt werden.

Nach dem erfindungsgemäßen Verfahren wird das Wertprodukt BHB wirtschaftlich, in guter Ausbeute und hoher Reinheit erhalten.

BHB dient für vielerlei Anwendungen, beispielsweise zur Herstellung von Alkydharzen und Lacken, als Polyolkomponente bei der Herstellung von Polyurethanen und zur Herstellung von Weichmachern und Emulgatoren.

Der zu seiner Herstellung verwendete 4-Hydroxybutyraldehyd kann gemäß EP-A 293 818 durch die Isomerisierung von 2-Butendiol-(1,4) oder alternativ, über die Hydroformylierung von Allylalkohol, wie in EP-A 150 943 beschrieben, gewonnen werden.

### Beispiele:

### Beispiel 1

176 g (2 Mol) 4-Hydroxybutyraldehyd, 500 g 30 Gew.%ige Formalinlösung (5 Mol Formaldehyd) und 3 g (0,03 Mol) Triethylamin wurden vermischt und erst 1 Std. bei 25°C, dann 1 Std. bei 40°C gerührt. Anschließend wurde die Reaktionsmischung mit 1000 g Wasser verdünnt und die resultierende Lösung bei 130°C und einem Druck von 90 bar kontinuierlich hydriert. Als Hydrierkatalysator wurde ein Kupfer auf Aluminiumoxid-Katalysator gemäß EP-A 44 444 verwendet, dessen Kupfergehalt, berechnet als CuO, 55 Gew.%, und dessen Aluminiumoxidgehalt, berechnet als Al₂O₃, 44,5 Gew.% betrugt. Die Katalysatorbelastung betrug 1,2 l Lösung/l Katalysator xh. Nach der destillativen Aufarbeitung des Hydrierproduktes wurden 201 g BHB (Siedepunkt (1 Torr): 200 bis 205°C; Schmelzpunkt: 87 bis 88°C), entsprechend einer Gesamtausbeute von 67 %, bezogen auf eingesetzten 4-Hydroxybutyraldehyd, isoliert.

### Beispiel 2

Die Aldolkondensation wurde wie in Beispiel 1 beschrieben durchgeführt. Anschließend wurde das im Reaktionsgemisch vorhandene Wasser bei vermindertem Druck (3 mbar, Sumpftemperatur: 50°C) weitgehend abdestilliert und der Rückstand in der dreifachen Menge Tetrahydrofuran gelöst. Die resultierende Lösung wurde bei 170°C und einem Druck von 90 bar kontinuierlich hydriert. Als Hydrierkatalysator wurde ein Katalysator gemäß DE-A 39 04 083 verwendet, der 66,8 Gew.% Kobalt, berechnet als CoO, 19,1 Gew.% Kupfer, berechnet als CuO, 7,1 Gew.% Mangan, berechnet als Mn₂O₃, 3,3 Gew.% Molybdän, berechnet als MoO₃, 3,5 Gew.% Phosphorsäure, berechnet als H₃PO₄ und 0,15 Gew.% Natrium, berechnet als Na₂O, enthielt. Der Katalysator wurde mit 1,9 l Lösung/l Katalysator xh belastet. Nach der destillativen Aufarbeitung des Hydrierproduktes wurde BHB in einer Gesamtausbeute von 54 %, bezogen auf eingesetzten 4-Hydroxybutyraldehyd, erhalten.

## Patentansprüche

1. Verfahren zur Herstellung von 2,2-Bis-hydroxymethyl-butandiol-(1,4) der Formel I 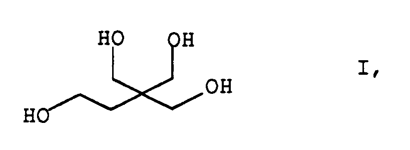 dadurch gekennzeichnet, daß man 4-Hydroxybutyraldehyd der Formel II 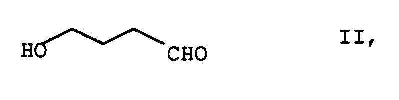 und/oder 2-Hydroxytetrahydrofuran der Formel III 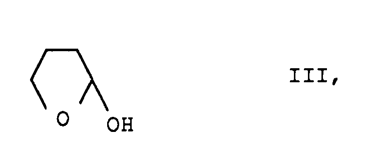 in Gegenwart eines basischen Katalysators mit Formaldehyd kondensiert, die erhaltene Reaktionsmischung anschließend katalytisch hydriert und aus dem Hydrieraustrag das 2,2-Bis-hydroxymethyl-butandiol-(1,4) isoliert.

2. Verfahren nach Anspruch 1, dadurch gekennzeichnet, daß man als basischen Katalysator ein tertiäres Amin verwendet.

3. Verfahren nach Anspruch 1, dadurch gekennzeichnet, daß man die Hydrierung in einem organischen Lösungsmittel oder in einem Gemisch aus organischem Lösungsmittel und Wasser durchführt.

4. Verfahren nach Anspruch 1, dadurch gekennzeichnet, daß das zu hydrierende Reaktionsgemisch einen Wassergehalt von 50 bis 90 Gew.% hat.

5. Verfahren nach Anspruch 1, dadurch gekennzeichnet, daß man die Hydrierung an einem heterogenen Hydrierkatalysator durchführt.

## Claims

1. A process for preparing 2,2-bishydroxymethyl-1,4-butanediol of the formula I 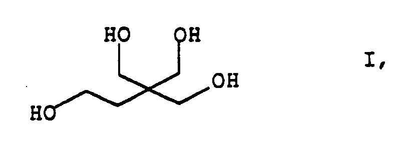 which comprises condensing 4-hydroxybutyraldehyde of the formula II 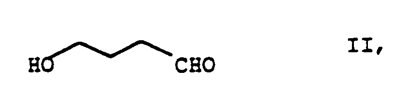 and/or 2-hydroxytetrahydrofuran of the formula III 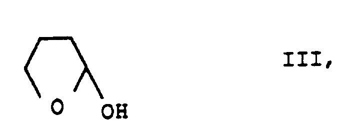 with formaldehyde in the presence of a basic catalyst, then catalytically hydrogenating the reaction mixture obtained and isolating the 2,2-bishydroxymethyl-1,4-butanediol from the mixture removed from the hydrogenation.

2. A process as claimed in claim 1, wherein the basic catalyst used is a tertiary amine.

3. A process as claimed in claim 1, wherein the hydrogenation is carried out in an organic solvent or in a mixture of organic solvent and water.

4. A process as claimed in claim 1, wherein the reaction mixture to be hydrogenated has a water content of from 50 to 90 % by weight.

5. A process as claimed in claim 1, wherein the hydrogenation is carried out on a heterogeneous hydrogenation catalyst.

## Revendications

1. Procédé de préparation du 2,2-bis-hydroxyméthylbutanediol-(1,4) répondant à la formule I 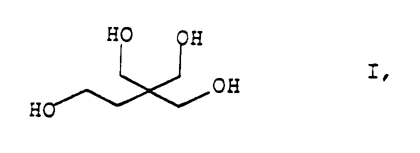 caractérisé en ce que l'on condense le 4-hydroxybutyraldéhyde répondant à la formule II 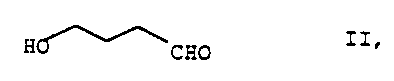 et/ou le 2-hydroxytétrahydrofuranne répondant à la formule III 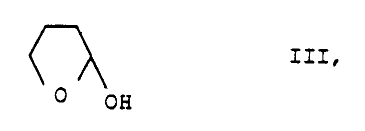 en présence d'un catalyseur basique, avec le formaldéhyde, on hydrogène ensuite le mélange réactionnel ainsi obtenu par voie catalytique et on isole le 2,2-bis-hydroxyméthylbutanediol-(1,4) du produit sortant de l'hydrogénation.

2. Procédé suivant la revendication 1, caractérisé en ce que l'on utilise une amine tertiaire à titre de catalyseur basique.

3. Procédé suivant la revendication 1, caractérisé en ce que l'on entreprend l'hydrogénation dans un solvant organique, ou dans un mélange constitué d'un solvant organique et d'eau.

4. Procédé suivant la revendication 1, caractérisé en ce que le mélange réactionnel à hydrogéner possède une teneur en eau de 50 à 90% en poids.

5. Procédé suivant la revendication 1, caractérisé en ce que l'on entreprend l'hydrogénation sur un catalyseur d'hydrogénation hétérogène.
